# EUROPEAN PATENT APPLICATION

(11) **EP 2 189 470 A1**
(43) Date of publication of application: **26.05.2010**
(21) Application number: 09011736.7
(22) Date of filing: 10.12.2002
(51) Int. Cl.: C07K 7/06, C07K 16/18, C12N 5/071, A61K 38/04, A61K 39/395, A61P 35/00, G01N 33/15, G01N 33/50, G01N 33/53, G01N 33/566

(54) **Tumor antigens**

(30) Priority: 10.12.2001 JP 2001376415
(62) Divisional of application: 02783816.8
(71) Applicant: Green Peptide Co., Ltd., Fukuoka 830-0018 (JP)
(72) Inventor: Itoh, Kyogo, Miyaki-gun Saga Prefecture 841-0205 (JP)
(74) Representative: Krauss, Jan

(57) **Abstract**

Tumor antigen peptides that induce CTL from peripheral blood mononuclear cells of a patient with prostate cancer in an HLA-A2-restricted or an HLA-A24-restricted manner were identified based on the amino acid sequences of PSA, PSM, and PSCA, which are proteins known as markers of prostate cancer. Also disclosed is a peptide having a function as a tumor antigen that is recognized by HLA-A2-restricted CTL or HLA-A24-restricted CTL, an antibody against the peptide, a compound having interaction with the peptide, an agent for inducing CTL that includes the peptide, a pharmaceutical composition that includes one or more thereof, a method for identifying a compound that has interaction with the peptide, a method for inducing CTL using the peptide, a method for measuring the peptide and the antibody, as well as a reagent kit used for the identification method or the measurement method.

SEQUENCE LISTING

<110>  ITOH, Kyogo

<120>  Tumor antigen

<130>  GP02-1018PCT

<150>  JP P2001-376415
<151>  2001-12-10

<160>  12

<170>  PatentIn version 3.1

<210>  1
<211>  9
<212>  PRT
<213>  Artificial

<220>
<223>  A peptide recognized by HLA-A24 restricted cytotoxic T lymphocyte
       s, which is designed based on the amino acid sequence of PSA

<400>  1

Cys Tyr Ala Ser Gly Trp Gly Ser Ile
1               5


<210>  2
<211>  10
<212>  PRT
<213>  Artificial

<220>
<223>  A peptide recognized by HLA-A24 restricted cytotoxic T lymphocyte
       s, which is designed based on the amino acid sequence of PSA

<400>  2

His Tyr Arg Lys Trp Ile Lys Asp Thr Ile
1               5                   10


<210>  3
<211>  9
<212>  PRT
<213>  Artificial

<220>
<223>  A peptide recognized by HLA-A24 restricted cytotoxic T lymphocyte
       s, which is designed based on the amino acid sequence of PSMA

<400>  3

Thr Tyr Ser Val Ser Phe Asp Ser Leu
1               5


<210>  4
<211>  9
<212>  PRT

<213>  Artificial

<220>
<223>  A peptide recognized by HLA-A24 restricted cytotoxic T lymphocyte
       s, which is designed based on the amino acid sequence of PSCA

<400>  4

Asp Tyr Tyr Val Gly Lys Lys Asn Ile
1               5


<210>  5
<211>  10
<212>  PRT
<213>  Artificial

<220>
<223>  A peptide recognized by HLA-A24 restricted cytotoxic T lymphocyte
       s, which is designed based on the amino acid sequence of PSCA

<400>  5

Lys Asn Ile Thr Cys Cys Asp Thr Asp Leu
1               5                   10


<210>  6
<211>  9
<212>  PRT
<213>  Artificial

<220>
<223>  A peptide recognized by HLA-A2 restricted cytotoxic T lymphocytes
       , which is designed based on the amino acid sequence of PSCA

<400>  6

Ala Leu Leu Met Ala Gly Leu Ala Leu
1               5


<210>  7
<211>  10
<212>  PRT
<213>  Artificial

<220>
<223>  A peptide recognized by HLA-A2 restricted cytotoxic T lymphocytes
       , which is designed based on the amino acid sequence of PSCA

<400>  7

Leu Leu Cys Tyr Ser Cys Lys Ala Gln Val
1               5                   10


<210>  8
<211>  10
<212>  PRT
<213>  Artificial

<220>

<223>  A peptide recognized by HLA-A2 restricted cytotoxic T lymphocytes
       , which is designed based on the amino acid sequence of PSA

<400>  8

Phe Leu Thr Pro Lys Lys Leu Gln Cys Val
1               5                   10


<210>  9
<211>  10
<212>  PRT
<213>  Artificial

<220>
<223>  A peptide recognized by HLA-A2 restricted cytotoxic T lymphocytes
       , which is designed based on the amino acid sequence of PSA

<400>  9

Val Ile Ser Asn Asp Val Cys Ala Gln Val
1               5                   10


<210>  10
<211>  30
<212>  PRT
<213>  Artificial

<220>
<223>  A oligopeptide recognized by HLA-A2 restricted cytotoxic T lympho
       cytes, which is designed based on the amino acid sequence of PSA

<400>  10

Phe Leu Thr Pro Lys Lys Leu Gln Cys Val Asp Leu His Val Ile Ser
1               5                   10                  15


Asn Asp Val Cys Ala Gln Val His Pro Gln Lys Val Thr Lys
            20                  25                  30


<210>  11
<211>  9
<212>  PRT
<213>  Artificial

<220>
<223>  A peptide recognized by HLA-A2 restricted cytotoxic T lymphocytes
       , which is designed based on the amino acid sequence of PSMA

<400>  11

Leu Leu His Glu Thr Asp Ser Ala Val
1               5


<210>  12
<211>  9
<212>  PRT
<213>  Artificial

<220>
<223>  A peptide recognized by HLA-A2 restricted cytotoxic T lymphocytes
       , which is designed based on the amino acid sequence of PSMA

<400>  12

Ala Leu Phe Asp Ile Glu Ser Lys Val
1               5

## Description

### TECHNICAL FIELD

The present invention relates to a peptide active as a tumor antigen peptide. More particularly, it relates to a peptide that is recognized by cytotoxic T-lymphocytes and/or induces cytotoxic T-lymphocytes in an HLA-A2-restricted or an HLA-A24-restricted manner, an antibody that is directed against the peptide, a compound having interaction with the peptide, an agent for inducing cytotoxic T-lymphocytes which comprises the peptide, a pharmaceutical composition comprising one or more thereof, a method for identifying a compound that has an interaction with the peptide, a method for inducing cytotoxic T-lymphocytes using the peptide, a method for measuring the peptide and the antibody, and a reagent kit.

### BACKGROUND

In eliminating cancer in vivo, the immune system and, in particular, cytotoxic T-lymphocytes involved in cell-mediated immunity play an important role. For instance, the infiltration of cytotoxic T-lymphocytes that demonstrate cytotoxicity against tumor cells has been observed in tumors in cancer patients (Non-Patent Reference 1). A target molecule for such tumor-specific cytotoxic T-lymphocytes, or so-called tumor antigen, was first discovered in melanoma. The tumor antigen that is generated in a tumor cell is degraded in the cell into a peptide that consists of 8 to 11 amino acids, which is a so-called tumor antigen peptide and binds to a molecule of human leukocyte antigen (hereinafter abbreviated as HLA) that is a major histocompatibility complex (hereinafter abbreviated as MHC) to be presented on the surface of the tumor cell. The cytotoxic T-lymphocyte recognizes the HLA molecule and tumor antigen peptide complex, releases a soluble factor or the like, for example, a cytokine such as interferon-γ, and then lyses the tumor cell. That is to say, the cytotoxic T-lymphocytes demonstrate a cytotoxic activity by recognizing tumor cells in an HLA-restricted manner.

HLA is a cell membrane antigen that is expressed in almost all eukaryotic cells. HLAs can be broadly divided into Class I antigens and Class II antigens. HLAs recognized together with an antigen peptide by a cytotoxic T-lymphocyte are considered Class I antigens. HLA Class I antigens are further classified into HLA-A, HLA-B, HLA-C, and the like, and in humans, different eukaryotic cells have different quantities of HLA-A, HLA-B, and HLA-C. In addition, genetic polymorphisms therefor have been reported. For instance, for HLA-A, there are such polymorphisms as A1, A2, A24, and A26; for HLA-B, there are such polymorphisms as B8, B27, and B46; and for HLA-C, there are such polymorphisms as Cw3 and Cw6. Thus, the types of HLA that each individual possesses are not necessarily identical. The HLA-A2 allele, which is one polymorphism of the HLA-A sub-region, is found in approximately 40% of Japanese, approximately 53% of Chinese, approximately 49% of North American Caucasians, approximately 38% of South American Caucasians, and approximately 23% of African Blacks (Non-Patent Reference 2). In addition, the HLA-A24 allele is found in approximately 60% of the Japanese population (for the most part, 95% of their genotype is A2402), approximately 20% of Caucasians, and approximately 12% of Africans.

When a cytotoxic T-lymphocyte recognizes the complex of an HLA Class I antigen and a tumor antigen peptide, it also recognizes the specific type of HLA. In addition, a motif (regular arrangement), which differs depending on the HLA type, is known to exist in the amino acid sequence of the tumor antigen peptides that bind to the HLA molecules. Since the peptides that bind to the HLA molecule differ depending on the type of HLA, it is necessary to select peptides that bind to each type of HLA in order to induce and/or activate an antigen-specific cytotoxic T-lymphocytes using a tumor antigen peptide.

In recent years, molecules that are involved in specific immunity, such as tumor rejection antigen genes and T-cell antigen receptors (T-cell receptors), have been identified in a variety of cancers, for instance, in melanoma, esophageal cancer, and the like. Specific immunotherapies using peptides are also being examined in advanced cancers and metastatic cancers (Non-Patent Reference 3, 4, 5, 6, 7, and 8).

Currently, in Europe and America, cancer vaccine therapies are being developed, wherein cytotoxic T-lymphocytes inside the body of a cancer patient are activated by the administration of a tumor antigen, and results from clinical studies have been reported for melanoma-specific tumor antigens. For example, by subcutaneously administrating melanoma antigen gp100 peptide, and intravascularly administrating interleukin-2 to melanoma patients, reduction of tumors was observed in 42% of the patients (Non-Patent Reference 9). Thus, an effective cancer therapy effect can be expected from a tumor antigen when it is used as a cancer vaccine.

However, when the diversity of cancers is considered, it is impossible to treat all cancers using a cancer vaccine consisting of only one type of tumor antigen. The diversity of cancer cells gives rise to diversity in the type or the amount of tumor antigens being expressed in the cancer cells. Furthermore, it has been reported that an immunotherapy using a plurality of peptides (multi-peptide based immunotherapy) is effective in cancer therapy (Non-Patent References 10, 11, and 12).

In addition, given that the type of tumor antigen peptide that functions in each individual differs due to polymorphisms of the HLA gene, it is important to identify a tumor antigen peptide that corresponds to each HLA in order to obtain a highly effective cancer therapy.

Obviously, a cancer vaccine therapy that activates cytotoxic T-lymphocytes using a single tumor antigen will bring some therapeutic effects for a cancer having this tumor antigen. However, in order to induce and/or activate antigen-specific cytotoxic T-lymphocytes, and also to obtain a highly effective therapeutic response to the diversity of cancers, it is important to discover and use numerous novel tumor antigens that correspond to the HLA-restricted and diverse nature of cancers.

The references cited in the description of the present technical background are listed below:
Non-Patent Reference 1: Archives of Surgery, 1990, Vol. 126, pp. 200-205;
Non-Patent Reference 2: HLA 1991, 1992, (UK: Oxford), Oxford Scientific Publications, Vol. 1, pp. 1065-1220;
Non-Patent Reference 3: Science, 1991, Vol. 254, pp. 1643-1647;
Non-Patent Reference 4: Journal of Experimental Medicine, 1996, Vol. 183, pp. 1185-1192;
Non-Patent Reference 5: Gomi, S. et al., Journal of Immunology, 1999, Vol. 163, pp. 4994-5004;
Non-Patent Reference 6: Proceedings of the National Academy of Sciences of the United States of America, 1995, Vol. 92, pp. 432-436;
Non-Patent Reference 7: Science, 1995, Vol. 269, pp. 1281-1284;
Non-Patent Reference 8: Journal of Experimental Medicine, 1997, Vol. 186, pp. 785-793;
Non-Patent Reference 9: Mature Medicine, 1998, Vol. 4, pp. 321-327;
Non-Patent Reference 10: Clinical Cancer Research, 2001, Vol. 7, pp. 3950-3962;
Non-Patent Reference 11: Journal of Clinical Oncology, 2001, Vol. 19, pp. 3836-3847; and
Non-Patent Reference 12: Nature Medicine, 1998, Vol. 4, pp. 328-332.

### DISCLOSURE OF THE INVENTION

In order to discover a novel peptide that acts as a tumor antigen peptide, for instance, a peptide that is recognized by cytotoxic T-lymphocytes and is useful for specific immunotherapy for a prostate cancer patient, the present inventor focused on three proteins known as markers of prostate cancer, i.e., prostate-specific antigen, prostate-specific membrane antigen, and prostate stem cell antigen. Then, among the partial peptides of these proteins, a tumor antigen peptide was identified that induces cytotoxic T-lymphocytes, in an HLA-A2-restricted or an HLA-A24-restricted manner, from peripheral blood mononuclear cells of a patient with a prostate cancer, and thus the present invention was completed.

One aspect of the present invention is a peptide having the amino acid sequence set forth in any one of SEQ ID NOS: 1 to 7 in the Sequence Listing.

A further aspect of the present invention is a peptide having the amino acid sequence set forth in any one of SEQ ID NOS: 1 to 7 in the Sequence Listing, wherein the peptide is recognized by a cytotoxic T-lymphocyte and/or induces a cytotoxic T-lymphocyte.

Still, a further aspect of the present invention is a peptide having the amino acid sequence set forth in any one of SEQ ID NOS: 1 to 5 in the Sequence Listing, wherein the peptide is recognized by a cytotoxic T-lymphocyte in an HLA-A24-restricted manner and/or induces a cytotoxic T-lymphocyte in an HLA-A24-restricted manner.

One additional aspect of the present invention is a peptide having the amino acid sequence set forth in SEQ ID NOS: 6 or 7 in the Sequence Listing, wherein the peptide is recognized by a cytotoxic T-lymphocytes in an HLA-A2-restricted manner and/or induces cytotoxic T-lymphocytes in an HLA-A2-restricted manner.

Additionally, the present invention is a medicament comprising at least one peptide having the amino acid sequence set forth in any one of SEQ ID NOS: 1 to 7 in the Sequence Listing.

In addition, one aspect of the present invention is a cancer vaccine comprising at least one peptide having the amino acid sequence set forth in any one of SEQ ID NOS: 1 to 7 in the Sequence Listing.

Furthermore, one aspect of the present invention is the aforementioned cancer vaccine that is used in a treatment of prostate cancer.

One further aspect of the present invention is an agent for inducing a cytotoxic T-lymphocyte, comprising at least one peptide having the amino acid sequence set forth in any one of SEQ ID NOS: 1 to 7 in the Sequence Listing.

One additional aspect of the present invention is a method for inducing a cytotoxic T-lymphocyte, comprising using at least one peptide having the amino acid sequence set forth in any one of SEQ ID NOS: 1 to 7 in the Sequence Listing.

In addition, one aspect of the present invention is a method for inducing a cytotoxic T-lymphocyte, comprising the steps of:
i) incubating an antigen-presenting cell that retains HLA-A24 with a peptide having the amino acid sequence set forth in any one of SEQ ID NOS: 1 to 5 in the Sequence Listing; and
ii) incubating an antigen-presenting cell that retains HLA-A2 with a peptide having the amino acid sequence set forth in SEQ ID NOS: 6 or 7 in the Sequence Listing; and
iii) stimulating a cell population that contain a precursor cell of the cytotoxic T-lymphocyte by using the cells obtained at steps i) and ii).

Furthermore, one aspect of the present invention is a method for inducing a cytotoxic T-lymphocyte, comprising the steps of:
i) incubating an antigen-presenting cell that retains HLA-A24 with a peptide having the amino acid sequence set forth in any one of SEQ ID NOS: 1 to 5 in the Sequence Listing; and
ii) stimulating a cell population that contain a precursor cell of the cytotoxic T-lymphocyte by using the cell obtained at step i).

Furthermore, one aspect of the present invention is a method for inducing a cytotoxic T-lymphocyte, comprising the steps of:
i) incubating an antigen-presenting cell that retains HLA-A2 with a peptide having the amino acid sequence set forth in SEQ ID NOS: 6 or 7 in the Sequence Listing; and
ii) stimulating a cell population that contain a precursor cell of the cytotoxic T-lymphocyte by using the cell obtained at step i).

In addition, one further aspect of the present invention is an antibody that immunologically recognizes the aforementioned peptides.

Moreover, one aspect of the present invention is a method for identifying a compound that interacts with the aforementioned peptides and/or an HLA-A24 molecule, and the compound at least enhances the recognition of the peptides by HLA-A24-restricted cytotoxic T-lymphocytes, wherein the method comprises using at least one substance selected from a group consisting of the aforementioned peptide and the aforementioned antibodies.

Moreover, one aspect of the present invention is a method for identifying compounds that interact with the aforementioned peptide and/or an HLA-A2 molecules, and at least enhance the recognition of the peptide by an HLA-A2-restricted cytotoxic T-lymphocyte, wherein the method comprises using at least one substance selected from a group consisting of the aforementioned peptides and the aforementioned antibodies.

In addition, one aspect of the present invention is a compound that is obtained by the aforementioned identification method.

In addition, a further aspect of the present invention is a compound that enhances the recognition of at least one of the aforementioned peptides by an HLA-A24-restricted cytotoxic T-lymphocyte.

One aspect of the present invention is, in addition, a compound that enhances the recognition of at least one of the aforementioned peptides by an HLA-A2-restricted cytotoxic T-lymphocyte.

Furthermore, one aspect of the present invention is a pharmaceutical composition comprising at least one substance selected from a group consisting of the aforementioned peptides, the aforementioned antibodies, and the aforementioned compounds.

In addition, one aspect of the present invention is a method for quantitatively or qualitatively measuring the aforementioned peptides or the aforementioned antibodies.

In addition, one aspect of the present invention is a reagent kit comprising at least one substance selected from a group consisting of the aforementioned peptides and the aforementioned antibodies.

In addition, one aspect of the present invention is a reagent kit that is used in the aforementioned method for inducing cytotoxic T-lymphocytes, the aforementioned identification method, or the aforementioned measurement method, wherein the reagent kit comprises at least one substance selected from a group consisting of the aforementioned peptides and the aforementioned antibodies.

### BRIEF DESCRIPTION OF THE DRAWINGS

Fig. 1 shows the result of the measurement of the anti-peptide antibodies in the serum of a prostate cancer patient, case 1.
Fig. 2 shows the result of the measurement of the anti-peptide antibodies in the serum of the prostate cancer patient, case 1.
Fig. 3 shows the result of the measurement of the anti-peptide antibodies in the serum of the prostate cancer patient, case 1.
Fig. 4 shows the result of the measurement of the anti-peptide antibodies in the serum of a prostate cancer patient, case 2.
Fig. 5 shows the result of the measurement of the anti-peptide antibodies in the serum of the prostate cancer patient, case 2.
Fig. 6 shows the result of the measurement of the anti-peptide antibodies in the serum of the prostate cancer patient, case 2.
Fig. 7A shows that each of PSA152-160 (SEQ ID NO: 1) and PSA248-257 (SEQ ID NO: 2) enhanced the production of interferon-γ from PBMC derived from an HLA-A24⁺ healthy subject, in reaction to an HLA-24⁺ prostate cancer cell PC93-A24. Fig. 7B shows that PBMC, which were derived from an HLA-A24⁺ healthy subject and co-cultured with PSA152-160 (SEQ ID NO: 1) or PSA248-257 (SEQ ID NO: 2), showed cytotoxic activity against PC93-A24. In Fig. 7B, * indicates that significance was established by the Student's t-test.
Fig. 8A shows that each of PSA152-160 (SEQ ID NO: 1) and PSA248-257 (SEQ ID NO: 2) enhanced the production of interferon-γ from PBMC derived from an HLA-A24⁺ prostate cancer patient, in reaction to HLA-24⁺ prostate cancer cell PC93-A24. Fig. 8B shows that PBMC, which were derived from an HLA-A24⁺ prostate cancer patient and co-cultured with PSA152-160 (SEQ ID NO: 1) or PSA248-257 (SEQ ID NO: 2), showed cytotoxic activity against PC93-A24. In Figs. 8A and 8B, * indicates that significance was established by the Student's t-test.
Fig. 9A shows that an antibody against PSA152-160 (SEQ ID NO: 1) or PSA248-257 (SEQ ID NO: 2) was detected in the serum of an HLA-A24⁺ prostate cancer patient. Fig. 9B shows that, when the serum of the HLA-A24⁺ prostate cancer patient, in which antibodies against PSA248-257 (SEQ ID NO: 2) were detected, was pre-adsorbed by PSA248-257 (SEQ ID NO: 2), the antibody titer thereof decreased. In Figs. 9A and 9B, * indicates that significance was established by the Student's t-test.
Fig. 10 shows that antibodies against PSA152-160 (SEQ ID NO: 1) or PSA248-257 (SEQ ID NO: 2) were detected in the serum of an HLA-A24⁺ healthy subject.
Fig. 11A shows that PSM624-632 (SEQ ID NO: 3) induced a CTL that recognizes HLA-24⁺ prostate cancer cells PC93-A24 from the PBMC of an HLA-A24⁺ prostate cancer patient. Fig. 11B shows that the production of IFN-γ against HLA-24⁺ prostate cancer cells PC93-A24, from the CTL that was induced from the PBMC of an HLA-A24⁺ prostate cancer patient by PSM624-632 (SEQ ID NO: 3), decreased. In Figs. 11A and 11B, * indicates that significance was observed by the Student's t-test.

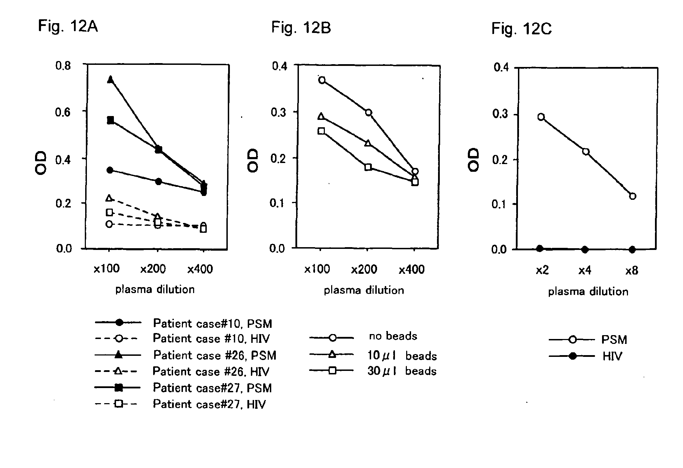

Fig. 12A shows that antibodies against PSM624-632 (SEQ ID NO: 3) were detected in the serum of an HLA-A24⁺ prostate cancer patient. Fig. 12B shows that, when the serum of an HLA-A24⁺ prostate cancer patient in which antibodies against PSA248-257 (SEQ ID NO: 2) were detected, was pre-adsorbed using beads coated with PSA248-257 (SEQ ID NO: 2), the antibody titer thereof decreased. Fig. 12C shows that antibodies against PSA248-257 (SEQ ID NO: 2) were detected in the eluate of beads that were coated with PSA248-257 (SEQ ID NO: 2) and used for adsorption of the serum of an HLA-A24⁺ prostate cancer patient.

### DETAILED DESCRIPTION OF THE INVENTION

The present invention claims priority from Japanese Patent Application 2001-376415, which is incorporated herein by reference.

In order to understand the present invention, the terminology used in the present specification will be explained first. A tumor antigen means a protein or peptide that a tumor cell posesses, which may be recognized by a tumor-specific cytotoxic T-lymphocyte (hereinafter may be abbreviated as CTL) and/or may induce a cytotoxic T-lymphocyte. In addition, a tumor antigen peptide means a peptide generated when the tumor antigen is degraded inside a tumor cell. This peptide may be recognized by tumor-specific cytotoxic T-lymphocytes and/or induce cytotoxic T-lymphocytes by binding to an HLA molecule and being presented on the surface of the cell.

Here, to "recognize" means that a recognizing entity discerns a to-be-recognized target from others and may bind to the recognized target. In particular, in the present specification, recognition of a tumor cell or a tumor antigen peptide by a cytotoxic T-lymphocyte means that the cytotoxic T-lymphocyte binds to a tumor antigen peptide presented by an HLA molecule via a T-cell antigen receptor. To "activate" means to further enhance or bring into action an entity or a state that has a given activity or effect. In particular, in the present specification, activation of a cytotoxic T-lymphocyte means that the cytotoxic T-lymphocyte generates, for example, interferon-γ (hereinafter abbreviated as IFN-γ) as a result of recognizing an antigen presented by an HLA molecule, or that the cytotoxic T-lymphocyte demonstrates a cytotoxic activity against the recognized target cell (also called target). To "induce" means to generate a given activity or effect in an entity or a state that substantially lacks the activity or the effect. In particular, in the present specification, to induce an antigen-specific cytotoxic T-lymphocyte means to cause a cytotoxic T-lymphocyte, that specifically recognizes a given antigen, to differentiate and/or proliferate in vitro or in vivo. In addition, in the present specification, a cytotoxic T-lymphocyte inducing agent means an agent that demonstrates an effect such as, for example, when CD8 positive T-cells that specifically recognize a given antigen is not present, or is present only at an extremely low ratio, is changed to a state where cytotoxic T-lymphocytes that recognize this antigen are present at an extremely high ratio. In the present specification, a long chain peptide among any peptides containing two or more amino acids bound to one another through a peptide bond or a modified peptide bond, can be called a polypeptide. For instance, in the present specification, proteins are also included in polypeptides. In addition, short chain peptides, also referred to as oligopeptides and oligomers, are simply called peptides. In the following, when an amino acid sequence is represented, it may be represented by one letter or by three letters.

Other technical and scientific terms used in the present specification have the meanings commonly understood by one of ordinary skill in the art to which the present invention pertains, unless otherwise defined. Reference is made herein to various methodologies known to those of ordinary skill in the art. Publications and other materials setting forth such known methodologies to which reference is made are incorporated herein by reference in their entireties.

Hereinafter, the present invention will be described in more detail for the various embodiments of the present invention. The following detailed description is illustrative, and its purpose is merely explanatory and does not limit the present invention in any way.

In the present invention, tumor antigen peptides (SEQ ID NOS: 1 to 7) were identified, which were derived from three known protein markers for prostate cancer, i.e., prostate-specific antigen (hereinafter abbreviated as PSA), prostate-specific membrane antigen (hereinafter abbreviated as PSMA), and prostate stem cell antigen (hereinafter abbreviated as PSCA). Concretely, peptides were designed and synthesized, which are conformable to HLA-A2-binding motifs or HLA-A24-binding motifs, based on the amino acid sequences of these proteins; and among these peptides, a peptide was identified which induces cytotoxic T-lymphocytes from peripheral blood mononuclear cells (hereinafter abbreviated as PBMC) of a patient with prostate cancer in an HLA-A2-restricted or an HLA-A24-restricted manner.

PSA is a protein (Accession Number: CAA28947) that is coded by a gene registered in the GenBank as Accession Number: X05332, and is a serine protease that belongs to the kinin-kallikrein family. PSA is expressed in a majority of prostate cancers, and two types of peptides derived from PSA, PSA-1 (SEQ ID NO: 8) and PSA-2 (SEQ ID NO: 9), have been reported to bind to HLA-A2 molecules and be recognized by cytotoxic T cells, as well as to induce HLA-A2-restricted cytotoxic T-lymphocytes (Correale, P. et al., Journal of The National Cancer Institute, 1997, Vol. 89, pp. 283-300). In addition, a 30mer oligopeptide (SEQ ID NO: 10) containing the amino acid sequences of PSA-1, PSA-3, and PSA-9 that has an HLA-A3-binding motif, has been reported to induce cytotoxic T-lymphocytes that recognizes HLA-A2-positive (hereinafter abbreviated as HLA-A2⁺) tumor cell that expresses PSA, from the PBMC derived from a healthy subject (Pierpaolo, C. et al., Journal of Immunology, 1998, Vol. 161, pp. 3186-3194).

PSMA is a protein (Accession Number: AAC83972) that is coded by a gene registered in the GenBank as Accession Number: AF007544, and has a pharmacological property of an N-acetylated α-linked acidic dipeptidase, and demonstrates hydrolysis activity to a substrate (Ruth, E. C. et al., Proceedings of the National Academy of Sciences of the United States of America, 1996, 93, pp.749-753). In addition, immunotherapy is under examination in a clinical study of prostate cancer, by inducing a cytotoxic T-lymphocytes using peptides derived from PSMA, which are PSM-P1 (SEQ ID NO: 11) and PSM-P2 (SEQ ID NO: 12), both of which are conformable to HLA-A2 binding motifs (Patricia, A.L. et al., Cancer Research, 2000, Vol. 60, pp. 829-833).

PSCA is a protein (Accession Number: AAC39607) that is coded by a gene registered in the GenBank as Accession Number: AF043498 and is a protein that belongs to the Thy-1/Ly-6 cell surface protein family. The mRNA of PSCA is known to be specifically expressed in the prostate, and to be over-expressed in prostate cancer (Robert, E.R. et al., Proceedings of the National Academy of Sciences of the United States of America, 1998, Vol. 95, pp. 1735-1740). In addition, a therapeutic effect of anti-PSCA antibody on prostate cancer has been observed in mice to which human prostate cancer was transplanted (Douglas, C.S. et al., Proceedings of the National Academy of Sciences of the United States of America, 2001, Vol. 98, pp. 2658-2663). In regard to PSCA, there is no report of a peptide that is recognized by cytotoxic T-lymphocytes in an HLA-A2-restricted manner or of a peptide that induces HLA-A2-restricted cytotoxic T-lymphocytes.

As mentioned before, expression of PSA, PSMA, and PSCA in prostate cancer was well-known; and with regard to PSA and PSMA, peptides derived therefrom which are conformable to HLA-A2 binding motifs were known to induce HLA-A2-restricted cytotoxic T-lymphocytes. However, among the seven types of peptides (SEQ ID NOS: 1 to 7) identified in the present invention, five types of peptides (SEQ ID NOS: 1 to 5) have an HLA-A24-binding motif and are peptides that are recognized by cytotoxic T-lymphocytes in an HLA-A24-restricted manner; and there are no reports regarding these peptides. In addition, the remaining two types of peptides (SEQ ID NOS: 6 and 7) have an HLA-A2-binding motif and are peptides that are recognized by cytotoxic T-lymphocytes in an HLA-A2-restricted manner; however, there are no reports regarding these peptides derived from PSCA.

Concretely, when PBMC derived from an HLA-A24-positive (hereinafter abbreviated as HLA-A24⁺) prostate cancer patient or an HLA-A24⁺ healthy subject were stimulated with a peptide having the amino acid sequence set forth in any one of SEQ ID NOS: 1 to 5 in the Sequence Listing, the production of IFN-γ from the PBMC was enhanced in a peptide-specific manner in reaction to C1 R-A2402 cells that were pulsed with peptides identical to the ones used in the stimulation.

In addition, a peptide having an amino acid sequence set forth in any one of SEQ ID NOS: 1 to 3 in the Sequence Listing enhanced the production of IFN-γ from PBMC derived from an HLA-A24⁺ prostate cancer patient or an HLA-A24⁺ healthy subject, in reaction to HLA-24⁺ prostate cancer cells. Furthermore, the PBMC, which were derived from an HLA-A24⁺ prostate cancer patient and stimulated with these peptides, recognized HLA-A24⁺ prostate cancer cells and demonstrated cytotoxicity against the cells.

Based on the above, it was revealed that a peptide having the amino acid sequence set forth in any one of SEQ ID NOS: 1 to 5 in the Sequence Listing, can induce CTL that recognize tumor cells in an HLA-A24-restricted manner and demonstrate cytotoxicity against the tumor cells, from PBMC derived from an HLA-A24⁺ prostate cancer patient or an HLA-A24⁺ healthy subject.

A peptide having an amino acid sequence set forth in SEQ ID NOS: 6 or 7 in the Sequence Listing enhanced the production of IFN-γ from PBMC in a peptide-specific manner, when the PBMC derived from an HLA-A2-positive (hereinafter abbreviated as HLA-A2⁺) prostate cancer patient or from an HLA-A2⁺ healthy subject were stimulated with either one of these peptides, in reaction to T2 cells that were pulsed with peptides identical to the one used in the stimulation.

In addition, the peptide having the amino acid sequence set forth in SEQ ID NO: 6 in the Sequence Listing, enhanced the production of IFN-γ from PBMC derived from an HLA-A2⁺ prostate cancer patient, in response to HLA-A2⁺ prostate cancer cells. The peptide having the amino acid sequence set forth in SEQ ID NO: 7 in the Sequence Listing, enhanced the production of IFN-γ from PBMC derived from an HLA-A2⁺ healthy subject, in response to HLA-A2⁺ prostate cancer cells. In addition, prostate cancer patient-derived PBMC stimulated with the peptide set forth in SEQ ID NO: 7 demonstrated cytotoxicity against HLA-A2⁺ prostate cancer cells.

Based on the above, it was revealed that a peptide having the amino acid sequence set forth in SEQ ID NO. 6 or 7 in the Sequence Listing, can induce CTL that recognizes tumor cells in an HLA-A2-restricted manner and demonstrate cytotoxic activity against the tumor cell, from PBMC derived from an HLA-A2⁺ prostate cancer patient or an HLA-A2⁺ healthy subject.

In addition, it was discovered that anti-peptide antibodies of immunoglobulin G (IgG) were present in the blood of a prostate cancer patient, which were raised against a peptide having the amino acid sequence set forth in any one of SEQ ID NOS: 2, 3, 4, 5, and 7 in the Sequence Listing. Thus, it was demonstrated that antibodies against a tumor antigen peptide are present in the blood of a cancer patient, and the presence or absence of these anti-peptide antibodies correlate with the recognition of the peptide by CTL and/or the induction of CTL. The correlation between the presence of IgG antibody against a tumor antigen peptide and the cancer vaccine effect of the peptide was first discovered in the present invention. Until the present invention, it had been reported that a natural antibody of IgG against polymorphic epithelial mucin (Muc1), which is a glycoprotein, was present in the serum of an early breast cancer patient, and that its presence and the prognosis of the disease were related (European Journal of Cancer, 1996, Vol. 32, pp. 1325-1331; Journal of Clinical Oncology, 2000, Vol. 18, pp. 574-583). Muc1 is expressed in a majority of adenocarcinomas and is used as a cancer-related antigen in the blood for monitoring the treatment of breast cancer patients and for the early detection of recurrence. In addition, Muc1 is also being examined as a vaccine for the immunotherapy of adenocarcinoma patients. Patients who have anti-Muc1 antibodies detected in the serum, have significantly increased survival rates compared to patients who do not have the antibodies. That is to say, it is suggested that the survival rate is higher in patients whose humoral immune response to Muc1 is observed. Based on the above, there is a possibility that a patient, who has IgG antibodies against the tumor antigen peptides that were obtained in the present invention, has a higher immune response to the tumor antigen peptides and a better prognosis, compared to a patient whose IgG antibodies against the peptides were not detected.

Thus, in the present invention, seven types of peptides were produced, which can be recognized by CTL and/or induce CTL in an HLA-A2-restricted manner or an HLA-A24-restricted manner. These peptides are the peptides having the amino acid sequences set forth in SEQ ID NOS: 1, 2, 3, 4, 5, 6, or 7 in the Sequence Listing. Since the peptides having the amino acid sequences set forth in any one of SEQ ID NOS: 1 to 5 in the Sequence Listing are recognized by HLA-A24-restricted antigen-specific CTL, they can be used as tumor antigen peptides that induce and/or activate the CTL. Since the peptide having the amino acid sequence set forth in SEQ ID NOS: 6 or 7 in the Sequence Listing are recognized by HLA-A2-restricted antigen-specific CTL, they can be used as tumor antigen peptides that induce and/or activate the CTL.

The aforementioned peptides may be used singly, or used in combination of two or more, in order to induce CTL and/or activate CTL. Since CTL is a group of several types of cells that recognize a variety of antigens, it is recommended to use these peptides preferably in combinations of two or more.

In addition, peptides that have one or more amino acids with mutation(s), such as deletion, substitution, addition, or insertion, in the peptides specified above, are also included within the scope of the present invention. Preferably, peptides having such mutation(s) are recognized by CTL, for instance, at least by HLA-A2-restricted CTL or HLA-A24-restricted CTL. Peptides having mutation(s) may be those that exist naturally or those into which mutation(s) have been introduced. The means for introducing mutation(s), such as deletion, substitution, addition, or insertion, are well-known, and the method by Ulmer (Science, 1983, Vol. 219, p. 666 et seq.), for instance, can be used. Insuring that the fundamental characteristics (physical properties, activity, or immunological activity and the like) of these peptides are not changed by the introduction of such mutation(s), as reciprocal substitution among, for example, homologous amino acids (polar amino acids, non-polar amino acids, hydrophobic amino acids, hydrophilic amino acids, positively charged amino acids, negatively charged amino acids, aromatic amino acids, and the like) is readily inferred. Furthermore, these usable peptides can be altered to the extent that no significant functional alteration is involved, such as modifying their constituent amino group or carboxyl group and the like.

The peptides of the present invention may be manufactured by general methods known in peptide chemistry. For instance, methods described in published technical books, such as Peptide Synthesis (Japan), Maruzen Co., Ltd., 1975, or Peptide Synthesis (USA), Interscience, 1996, may be cited as examples; however, many known methods other than these can be used.

In terms of purification and collection of the peptides of the present invention, it is possible to purify and collect them by using gel chromatography, ion column chromatography, affinity chromatography, or the like, or combinations thereof, or by a fractionation means based on a difference in solubility using ammonium sulfate, alcohol, and the like or combinations thereof. A method can be used in which peptides are specifically adsorbed and collected by using polyclonal antibodies or monoclonal antibodies, which can be prepared against the peptides based on their amino acid sequences.

The antibody of the present invention is an antibody that recognizes or binds to the aforementioned peptides of the present invention. Antibodies are not limited in particular, as long as they immunologically recognize or bind to the peptides. The antibodies may be a natural antibodies or antibodies prepared by using antigens. When preparing the antibodies, the antigens can be the aforementioned peptides or fragments thereof. The fragments are constituted by preferably having at least five, more preferably eight to ten amino acids each. Whether or not the binding or recognition occurs can be determined by the well-known antigen-antibody-binding reaction.

Any antibody preparation method well-known in the art can be used to produce the antibodies. For instance, they can be obtained by administering to an animal a peptide of the present invention with or without linking such to a carrier, in the presence or absence of an adjuvant, to induce immunity, such as a humoral response and/or a cell-mediated response. Any carrier can be used as long as it exerts no harmful effect by itself onto the host and enhances antigenicity. For example, cellulose, polymeric amino acids, albumin, keyhole limpet hemocyanin (KLH), and the like can be given as examples, but it is not limited thereto. As examples of adjuvants, Freund complete adjuvant (FCA), Freund incomplete adjuvant (FIA), Ribi (MPL), Ribi (TDM), Ribi (MPL+TDM), *Bordetella pertussis* vaccine, muramyl dipeptide (MDP), aluminum adjuvant (ALUM), and combinations thereof can be given. Mouse, rat, rabbit, goat, horse, and the like are suitable animals for immunization,.

The polyclonal antibodies can be obtained from the sera of the animals that were immunized with the antigen, by a method well-known in the art for collecting an antibody. As a preferable means, the immuno-affinity chromatography method can be given.

Monoclonal antibodies can be produced by collecting antibody-producing cells (for instance, lymphoid cells derived from the liver or the lymph node) from the animals subjected to the aforementioned immunization, followed by introducing a well-known transformation technique with a permanently proliferating cell line (a myeloma strain, such as the P3-X63-Ag81 strain or the like). For example, an antibody-producing cell can be fused with a permanently proliferating cell by methods that are well-known to one skilled in the art to create a hybridoma, which can then be cloned, followed by selecting hybridoma that produces an antibody that recognizes specifically the aforementioned peptides; and then the antibody is collected from a cultured solution of the hybridoma.

The polyclonal or monoclonal antibodies thus obtained, which can recognize and bind to the aforementioned peptide, can be used for a purification of the peptide, reagent, or labeling marker for the peptide.

The aforementioned peptides and antibodies that immunologically recognize each of these peptides provide, independently or in combinations thereof, an effective means for identifying a substance that enhances the recognition of the peptides by CTL. The identification method can be established using pharmaceutical screening systems that are well-known in the art. For instance, a substance that enhances recognition of the peptides of the present invention by CTL can be screened by stimulating CTL with antigen-presenting cells which have been pulsed with a tumor antigen peptide, followed by establishing an experimental system to measure recognition of the tumor antigen peptide by the CTL and/or activation of the CTL, and examining the test substance. Examples of antigen-presenting cells, include cells and cell strains that retain HLA-A2 molecules and/or HLA-A24 molecules, such as cell strains that retain HLA-A2 and/or HLA-A24 molecules. More concretely, T2 cells (HLA-A2⁺) and the like, can be given as examples. Otherwise, even cells that do not possess HLA-A2 molecules or HLA-A24 molecules can be used by genetically introducing thereto HLA-A2 cDNA or HLA-A24 cDNA so that HLA-A2 molecules or HLA-A24 molecules are expressed on the cell surface. Pulsing the tumor antigen to antigen-presenting cells can be performed by incubating the antigen-presenting cells with tumor antigen by any suitable method.

Examples of CTL include, for example, an HLA-A2-restricted CTL strain or an HLA-A2-restricted CTL line, for example, OK-CTL (Non-Patent Reference 5) and the like, is used. An HLA-A24-restricted CTL strain or an HLA-A24-restricted CTL line, can be KE4-CTL (International Journal of Cancer, 1999, Vol. 81, pp. 459-466) and the like. Recognition of the tumor antigen peptide by the CTL and/or activation of the CTL can be determined by measuring the quantity of IFN-γ production from the CTL as an indicator. This experimental system is to describe one identification method, and the identification method of the present invention is not restricted thereby.

KE4-CTL is an HLA-A24-restricted CTL cell strain that has been established from tumor-infiltrating lymphocytes of an esophageal cancer patient. Although the KE-4 cell is an esophageal cancer cell, it has been revealed that PAP (prostate acid phosphatase), a prostate cancer marker, is also expressed in this cell (Journal of Urology, 2001, Vol. 166, pp. 1508-1513). This shows that PSA, PSMA, and PSCA, from which the peptide of the present invention was derived, can also be expressed in KE-4 cells and, therefore, that KE-4CTL can recognize the peptide of the present invention presented by the HLA-A24 molecule. Therefore, KE-4CTL can be used for the identifying a substance that enhances the recognition of the peptides by CTL.

The present invention also includes compounds obtained by the aforementioned identification method. The compounds may be compounds that interact with a peptide of the present invention, for example, compounds having any one of the amino acid sequences set forth in SEQ ID NOS: 1 to 5 in the Sequence Listing, and/or with HLA-A24 to enhance the recognition of the peptide by HLA-A24-restricted CTL. Otherwise, the invention could include compounds that interact with a peptide, for example, a peptide having the amino acid sequences set forth in SEQ ID NOS 6 or 7 in the Sequence Listing, and/or with HLA-A2 to enhance the recognition of the peptide by HLA-A2-restricted CTL. A compound screened in such a manner can be prepared as a pharmaceutical composition by selecting it while taking into account the balance between biological usefulness and toxicity.

The peptides of the present invention can be used as tumor antigen peptides in order to induce and/or activate antigen-specific CTL in an HLA-A2-restricted manner or in an HLA-A24-restricted manner. That is to say, a medicament that contains one or more peptides selected from the aforementioned peptides, an agent for inducing CTL that contains one or more peptides selected from the aforementioned peptides, as well as a method for inducing CTL that comprises using one or more peptides selected from the aforementioned peptides, are also included within the scope of the present invention.

The aforementioned method for inducing CTL includes, as one embodiment thereof, a step of pulsing a peptide of the present invention to an antigen-presenting cell, and a step of stimulating a cell group that contains a CTL precursor cell using the antigen-presenting cells that have been obtained from the aforementioned step. Cells that retain HLA-A2 molecules and/or HLA-A24 molecules, for example, cell strains that retain HLA-A2 and/or HLA-A24 molecules, can be used as antigen-presenting cells. More concretely, T2 cells and the like can be given as cell strains that retain HLA-A2 molecules. Otherwise, cells can also be produced by genetically introducing HLA-A2 cDNA or HLA-A24 cDNA by any suitable method to make the cells express an HLA-A2 molecule or an HLA-A24 molecule on their surface. Pulsing the peptide to antigen-presenting cells can be performed by incubating the antigen-presenting cells with tumor antigen peptide by the methods known in the art. The cell group containing a CTL precursor cell can be, for instance, a peripheral blood cell, and more preferably a peripheral blood mononuclear cell. The stimulation of a cell group can be performed by using each of the HLA-A24⁺ antigen presenting cells and HLA-A2⁺ antigen presenting cells singly to which peptides have been pulsed, or by using both simultaneously.

In addition, pharmaceutical compositions can be provided which contain at least one of the following: the peptide of the present invention; antibodies that immunologically recognize the peptides; compounds that interact with the peptides and/or HLA-A2 molecules and enhance the recognition of the peptides by the CTL; or compounds that interact with the peptides and/or HLA-A24 molecules and enhance the recognition of the peptides by the CTL, when used alone or in combination.

The aforementioned medicament, the aforementioned pharmaceutical composition, the aforementioned agent for inducing CTL, and the aforementioned method for inducing CTL are useful, for instance, in the treatment of cancers, such as, the treatment of prostate cancers. The HLA-A2 allele, which is one polymorphism of the HLA-A sub-region, is found in approximately 40% of Japanese, approximately 53% of Chinese, approximately 49% of North American Caucasians, approximately 38% of South American Caucasians, and approximately 23% of African Blacks (Non-Patent Reference 2). The HLA-A24 allele is found in approximately 60% of the Japanese population (for the most part, 95% of its genotype is A2402), approximately 20% of Caucasians, and approximately 12% of Africans. Based on the above, the medicament, the pharmaceutical composition, the agent for inducing CTL, and the method for inducing CTL, which are included in the present invention, can be expected to be effective on a multitude of patients.

Concretely, for instance, medicaments comprising a peptide of the present invention and pharmaceutical compositions containing a peptide of the present invention can be used as so-called cancer vaccines. The term cancer vaccine as used herein means a drug that selectively damages tumor cells by inducing and/or enhancing a specific immune response against the tumor cells. The dosage thereof can be determined with appropriate modifications according to the extent of recognition of peptides by CTL; in general, it is between 0.01 and 100 mg/day/adult human, or preferably between 0.1 and 10 mg/day/adult human as an active principle. This can be administered from once every few days to every few months. Administration can be carried out according to well-known methods for administrating a peptide for medical use, such as subcutaneously, intravenously, or intramuscularly. In order to induce and/or enhance the immune response during administration, the peptide of the present invention can be used, in the presence or absence of an appropriate adjuvant, with or without linking to a carrier. The carrier is not limited as long as it exerts no harmful effect by itself onto the human body and is capable of enhancing antigenicity; cellulose, polymeric amino acids, albumin, and the like can be given as examples of carriers. Adjuvants can be those used in general for peptide vaccine inoculation, and a Freund incomplete adjuvant (FIA), aluminum adjuvant (ALUM), *Bordetella pertussis* vaccine, mineral oil, and the like can be given as examples. In addition, the formulation can be suitably selected by applying a suitable well-known method for formulating a peptide.

Otherwise, an effective cancer vaccine effect can be obtained also by collecting a fraction of mononuclear cells from the peripheral blood of a patient, incubating them with the peptide of the present invention, and then returning the fraction of mononuclear cells in which induction of CTL and/or activation of CTL was observed, into the blood of the patient. Culture conditions, such as mononuclear cell concentration, concentration of the peptide of the present invention, culture time, and the like, can be determined by simply repeating experiments. A substance having a capability to enhance the growth of lymphocytes, such as interleukin-2, may be added during culturing.

The peptides of the present invention can be effectively used alone or in combination as a cancer vaccine. Since it has been reported that multi-peptide based immunotherapies are effective (Non-Patent References 9, 10, and 11), and since the CTL of a cancer patient are a cell population comprising plural cells that recognize a plurality of tumor antigens, higher effectiveness may be obtained by using a plurality of types of peptides in combination as a cancer vaccine rather than by using one type of peptide. In particular, in a patient having both HLA-A24 and HLA-A2, a high effectiveness can be obtained by using a peptide that is recognized by an HLA-A24-restricted CTL and a peptide that is recognized by an HLA-A2-restricted CTL in combination for use as a cancer vaccine. In addition, in such a case, peptides selected from the peptides of the present invention can be used in combination; otherwise, at least one type of peptide selected therefrom and at least one type of tumor antigen peptide selected from well-known tumor antigen peptides may be used in combination. As examples of the well-known tumor antigen peptides, a tumor antigen peptide derived from the Ick gene or the src gene (International Patent Publication No. WO01/11044), tumor antigen peptide derived from the SART-1 gene (International Journal of Cancer, 1999, Vol. 81, pp. 459-466), tumor antigen peptide derived from the SART-3 gene (Cancer Research, 1999, Vol. 59, pp. 4056-4063), as well as tumor antigen peptide derived from cyclophilin B gene (Non-Patent Reference 5), and the like can be given, though the present invention is not limited thereto.

The peptides of the present invention and the antibodies that immunologically recognize the peptides can be used singly as diagnostic markers or reagents and the like. When used as reagents, they may contain substances such as a buffering solution, salt, stabilization agent, and/or antiseptic agent. In addition, the present invention also provides a reagent kit comprising one or more containers that are filled with one or more kinds of these reagents. Furthermore, for the formulation, formulation means that are well-known for a peptide or antibody can be used. These reagents and reagent kits can be used in the aforementioned identification method, the aforementioned method for inducing CTL, or the quantitative or qualitative measurement of the peptide of the present invention. The measurement method can be established using methods that are well-known to those skilled in the art. Radioimmunoassay, competitive binding assay, western blot analysis, enzyme-linked immunosorbent assay (ELISA), and the like can be given as examples of the measurement methods.

In addition, regarding a blood sample derived from a patient, quantitative or qualitative measurement of an anti-peptide antibody against the peptide of the present invention can be performed using the aforementioned reagents or reagent kits. For the measurement, serum or plasma can be used as the blood sample derived from a patient. As mentioned before, since there seems to be a relationship between the presence of an anti-peptide antibody in the blood of a cancer patient and the cancer vaccine effect of the peptide that is recognized by the antibody, it is believed that the assessment of the effectiveness of the peptide as a cancer vaccine becomes possible by measuring the anti-peptide antibody.

### EXAMPLES

The present invention will be described more concretely in the following by showing examples; however, the present invention is not limited to these examples.

### Example 1

### (Identification of a peptide recognized by HLA-A24-restricted CTL)

HLA-A24-binding motifs were obtained by searching the literature, then 9-mer or 10-mer peptides that are conformable to these motifs were designed and synthesized by well-known methods in the art based on the amino acid sequences of three proteins that are known as prostate cancer markers, i.e., PSA, PSMA, and PSCA. The purity of all the synthesized peptides was 70% or greater.

The ability of the synthesized peptide to induce HLA-A24-restricted CTL from the PBMC that was prepared from a prostate cancer patient was examined. The PBMC were prepared from blood by a method of the art. 1x10⁵ PBMC were cultured with 10 µM of each peptide in each well of a U-shaped 96-well micro-culture plate (produced by Nunc) to which 200 µl of culture medium (comprising 45% RPMI-1640 culture medium, 45% AIM-V^{®} culture medium (Invitrogen), 100 U/ml of IL-2, 0.1 mM of MEM non-essential amino acid solution (Invitrogen), and 10% FCS) was added, at 37°C under conditions of 5% CO₂-95% air. At 4 days and 7 days of culturing, half of the culture medium was removed and replaced with a culture medium having the aforementioned composition containing the peptide equivalent to the peptide that was added in the beginning. At 10 days of culturing, the cells were collected, washed, and used as effector cells (E).

Meanwhile, each synthesized peptide (10 µM) was incubated with C1R-A2401 cells (HLA-A24⁺) for 2 hours at 37°C under conditions of 5% CO₂-95% air, to make the peptide of interest bind to the HLA-A24 molecule expressed on the cell surface. C1R-A2401 cells thus pulsed with each of peptide were used as target cells (T).

The effector cells were equally divided, and mixed with a variety of target cells, followed by incubating for 18 hours at 37°C under conditions of 5% CO₂-95% air. After incubation, 100 µl of supernatant was collected, and the amount of IFN-γ produced was measured by ELISA. In so doing, the effector cells were paired with the target cells in such a way that the peptide used to induce the effector cells and the peptide pulsed to C1R-A2401 cells were the same. In addition, a peptide derived from the human immunodeficiency virus (hereinafter abbreviated as HIV) was used as a control, and the quantity of IFN-γ produced by CTL that recognized C1R-A2401 cells pulsed with the HIV peptide was taken as a background and subtracted from each measurement value. Each measurement value (NET) that has been calculated was statistically processed by the Student's t-test, and those with a P value of 0.05 or below were considered to be significant. In addition, a peptide that enhanced IFN-γ production with significance by 50 pg/ml NET or more was assessed as positive.

As a result, PSA152-160 (SEQ ID NO: 1) and PSA248-257 (SEQ ID NO: 2), which were derived from PSA, PSM624-632 (SEQ ID NO: 3), which was derived from PSMA, as well as PSCA76-84 (SEQ ID NO: 4) and PSCA82-91 (SEQ ID NO: 5), which were derived from PSCA, enhanced the production of IFN-γ from PBMC of prostate cancer patients in 2 out of 14 cases, 4 out of 13 cases, 5 out of 14 cases, 1 out of 12 cases, and 2 out of 12 cases, respectively. That is to say, it was revealed that these peptides induced HLA-A24-restricted CTL from the PBMC of prostate cancer patients. In addition, the numbers MMM-NNN in the peptide names mean that the peptide has the amino acid residues from the position MMM to the position NNN within the amino acid sequence of the protein from which the peptide was derived.

### Example 2

### (Cytotoxic activity of CTL induced by peptides)

The cytotoxic activity of CTL induced from the PBMC of an HLA-A24⁺ prostate cancer patient by PSM624-632 (SEQ ID NO: 3) was examined in the ⁵¹Cr release test using prostate cancer cells. First, PBMC from two patients with prostate cancer were cultured for 10 days and stimulated as in Example 1, and then further cultured for 10 days prior to use as effector cells. The cytotoxic activity of the effector cells against HLA-A24⁺ target cells was measured by a standard 6-hour ⁵¹Cr release test at an E/T ratio of 10:1 or 20:1, and the results obtained were expressed as the percentage of specific lysis. PC93-A24 cells resulting from the introduction and expression of the HLA-A24 gene in HLA-A24⁻ prostate cancer cells (PC93), methods known in the art, were used as target cells. In addition, the HLA-A24⁻ prostate cancer cells were used as controls.

As a result, the PBMC derived from the prostate cancer patients, which were stimulated with PSM624-632 (SEQ ID NO: 3), specifically lysed prostate cancer cells that express HLA-A24 in both cases (Table1). This makes it clear that PSM624-632 (SEQ ID NO: 3) induces HLA-A24-restricted tumor-specific cytotoxic T-lymphocytes from the PBMC of cancer patients.

**Table 1**

| PBMC donors | Cytotoxic activity (% lysis) | | | |
|---|---|---|---|---|
| | HLA-A24⁻ prostate cancer cell | | HLA-A24⁺ prostate cancer cell | |
| | E/T=10 | E/T=20 | E/T=10 | E/T=20 |
| Prostate cancer patient 1 | 6.6 | 6.1 | 16.1 | 19.9 |
| Prostate cancer patient 2 | 3.5 | 5.6 | 18.0 | 17.8 |

### Example 3

### (Measurement of anti-peptide antibody in the sera of prostate cancer patients)

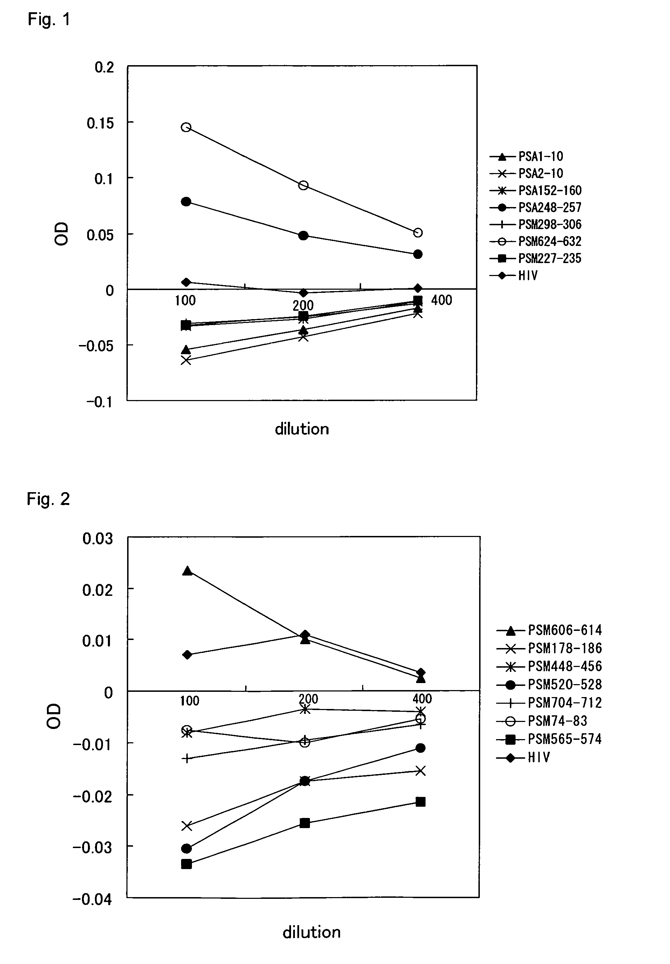

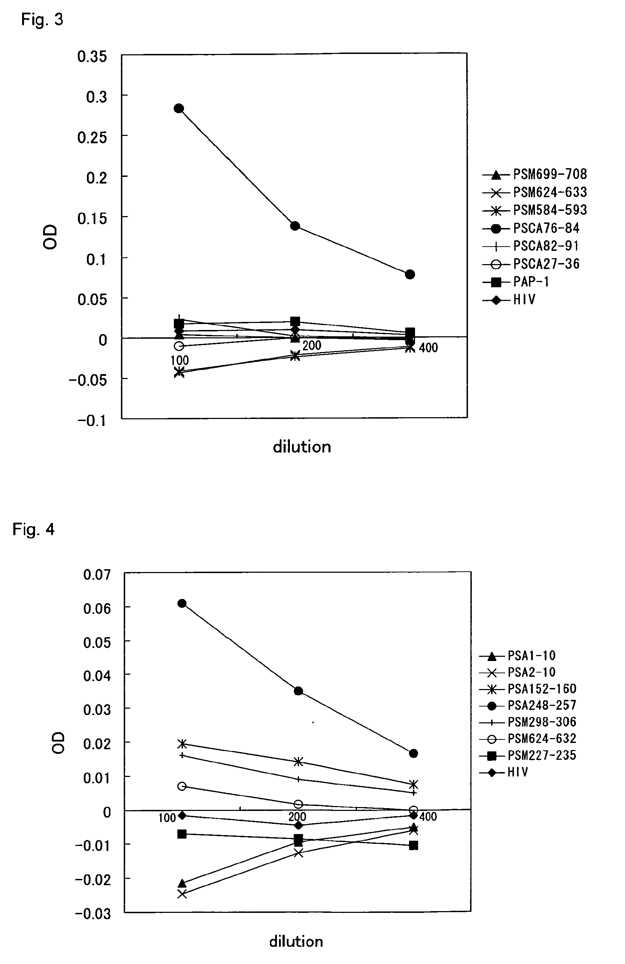

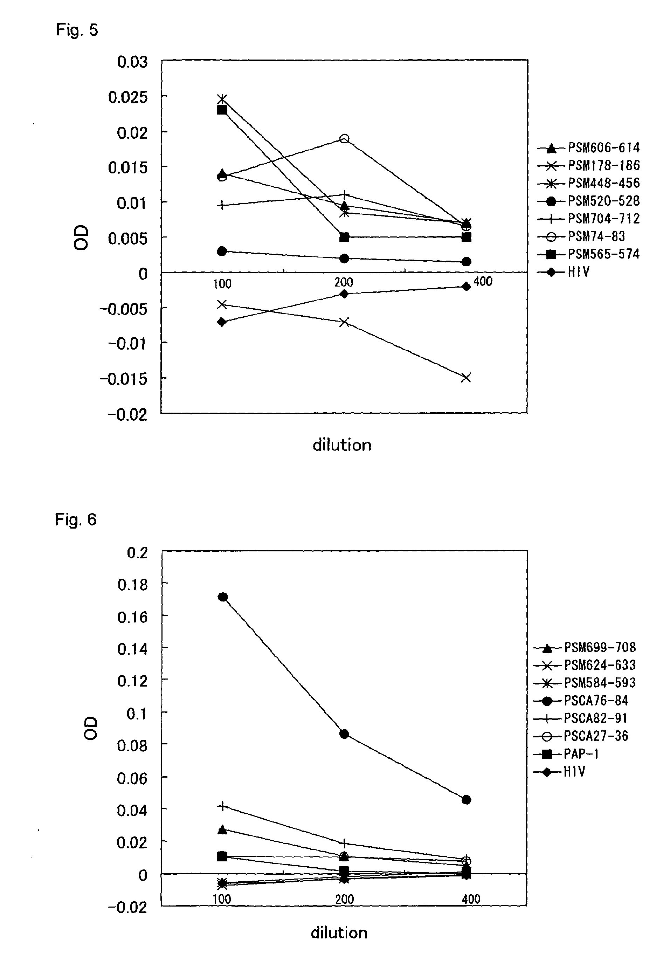

Anti-peptide antibodies of immunoglobulin G (IgG) in the sera of prostate cancer patients were measured by ELISA as described below. As a result, antibodies against PSA248-257 (SEQ ID NO: 2), PSM624-632 (SEQ ID NO: 3), PSCA76-84 (SEQ ID NO: 4), and PSCA82-91 (SEQ ID NO: 5), which are the peptides that induced the HLA-A24-restricted CTL from the PBMC of prostate cancer patients, were detected in 5, 1, 4, and 2 out of 10 patients, respectively. However, antibodies against PSA152-160 (SEQ ID NO: 1) were not detected. Meanwhile, antibodies against PSA1-10 and PSA2-10 derived from PSA, PSM298-306, PSM227-235, PSM606-614, PSM178-186, PSM448-456, PSM520-528, PSM704-712, PSM74-83, PSM565-574, PSM699-708, PSM624-633, and PSM584-593 derived from PSMA, as well as PSCA27-36, which are the peptides that did not induce HLA-A24-restricted CTL, were not detected. Exemplary results are shown in Fig. 1 to Fig. 6. The results for Patient Case 1 are shown in Fig. 1 to Fig. 3, and the results for Patient Case 2 are shown in Fig. 4 to Fig. 6. In Patient Case 1, antibodies against each of PSA248-257 (SEQ ID NO: 2), PSM624-632 (SEQ ID NO: 3), and PSCA76-84 (SEQ ID NO: 4) were detected in the sera (Fig. 1 and Fig. 3). Further, in Patient Case 2, antibodies against each of PSA248-257 (SEQ ID NO: 2) and PSCA76-84 (SEQ ID NO: 4) were detected (Fig. 4 and Fig. 6).

These results demonstrate that antibodies against a tumor antigen peptide are present in the blood of a cancer patient, and the presence or absence of the anti-peptide antibodies and the recognition of the peptides by CTL and/or induction of CTL are correlated.

The anti-peptide antibodies (IgG) were measured by ELISA. Each aforementioned peptide (20 µg/well) was immobilized in a 96-well Nunc Covalink flat-bottomed plate (Fisher Scientific) using disuccinimidyl suberate (PIERCE) by following the product instructions. In so doing, wells onto which the peptides were not immobilized were included in the plate as a background. The plate treated in this way was blocked with Blockace (Yukijirushi), and washed with 0.05% Tween 20-PBS, prior to adding a serum or blood plasma sample that was diluted with 0.05% Tween 20-Blockace at volumes of 100 µl/well to the plate. After 2-hours incubation at 37°C, the plate was washed with Tween 20-PBS, and then rabbit anti-human IgG antibody (γ chain-specific antibody; DAKO) that was diluted to 1:1000 was added to the plate followed by incubating for 2 additional hours at 37°C. The plate was then washed 9 times, and 100 µl of horseradish peroxidase dextran polymer (EnVision, DAKO) linked to a goat anti-rabbit Ig antibody that was diluted to 1:100 was added to each well followed by incubating for 40 minutes at room temperature. After washing, 100 µl of tetramethylbenzidine substrate solution (KPL) was added; thereafter, the reaction was stopped by adding 1 M phosphoric acid, and the optical density (OD) at a wavelength of 450 nm was measured. The value obtained by subtracting the optical density of the well where the peptide was not immobilized from the optical density of the well where each peptide was immobilized was represented as the amount of each anti-peptide antibody in the Figures.

### Example 4

### (Examination of the abilities of PSA152-160 (SEQ ID NO: 1) and PSA248-257 (SEQ ID NO: 2) to induce a cytotoxic T-lymphocyte)

PBMC were prepared from an HLA-A24⁺ healthy subject and HLA-A24⁺ prostate cancer patient, and then induction of CTL that react to prostate cancer by PSA152-160 (SEQ ID NO: 1) and PSA248-257 (SEQ ID NO: 2) was examined. PBMC were used after culturing with 10 µg/ml each of PSA152-160 (SEQ ID NO: 1) and PSA248-257 (SEQ ID NO: 2) for 12 days by the same method as in Example 1. C1R-A2401 cells (HLA-A24⁺) that were pulsed with each peptide (10 µg/ml) by the same method as in Example 1 were used as target cells. The PBMC and target cells were mixed and incubated as in Example 1, and the amount of IFN-γ in the culture supernatant was measured.

The results show that the PBMC of a prostate cancer patient that were incubated with each of PSA152-160 (SEQ ID NO: 1) and PSA248-257 (SEQ ID NO: 2) had enhanced production of IFN-γ in 3 out of 6 cases and 4 out of 7 cases, respectively. Taken together with the results from the separate cases in Example 1, PSA152-160 (SEQ ID NO: 1) and PSA248-257 (SEQ ID NO: 2) enhanced the production of IFN-γ in 5 out of 20 cases and 8 out of 20 cases respectively. In addition, the PBMC from 1 out of 5 healthy cases, which were incubated with each of these peptides, recognized the C1 R-A2401 cells that were pulsed with the corresponding peptide and enhanced the production of IFN-γ.

Next, using PSA152-160 (SEQ ID NO: 1) and PSA248-257 (SEQ ID NO: 2), induction of CTL that recognize prostate cancer cells and exhibit cytotoxic activity was examined for the PBMC that were derived from healthy subjects. The PBMC used in the present example were derived from a healthy subject whose PBMC showed peptide-specific production of IFN-γ by PSA152-160 (SEQ ID NO: 1) or PSA248-257 (SEQ ID NO: 2). PBMC were cultured with 10 µg/ml each of PSA152-160 (SEQ ID NO: 1) or PSA248-257 (SEQ ID NO: 2) for 12 days, in the same manner as described above. Prostate cancer cells PC93-A24, which were created in Example 2 and used as target cells, were incubated with the aforementioned cultured PBMC, and then the amount of IFN-γ in the culture supernatant was measured (see Example 1). In addition, the aforementioned cultured cells were cultured for 14 additional days, and the cytotoxic activity against the PC93-A24 cells was measured by the ⁵¹Cr release test (see Example 2).

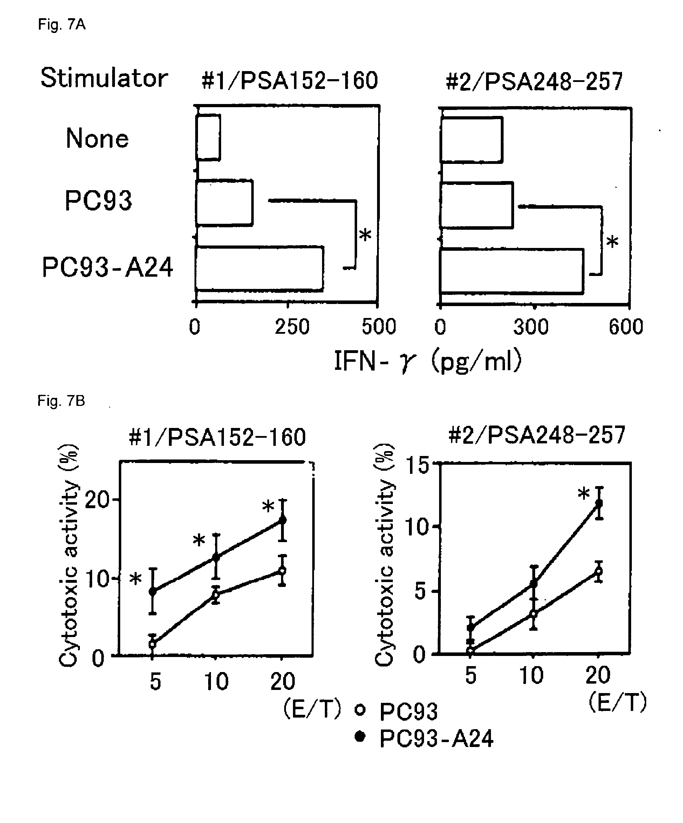

The results are shown in Fig. 7A and Fig. 7B. The PBMC derived from a healthy subject, which were stimulated with PSA152-160 (SEQ ID NO: 1) or PSA248-257 (SEQ ID NO: 2), respectively produced high levels of IFN-γ in response to PC93-A24 cells with significance (Fig. 7A). In addition, the PBMC derived from a healthy subject, which were stimulated with PSA152-160 (SEQ ID NO: 1) or PSA248-257 (SEQ ID NO: 2), exhibited cytotoxic activity against PC93-A24 cells and lysed these cells. The cytotoxic activity was significant in comparison to the activity against PC93 cells (Fig. 7B).

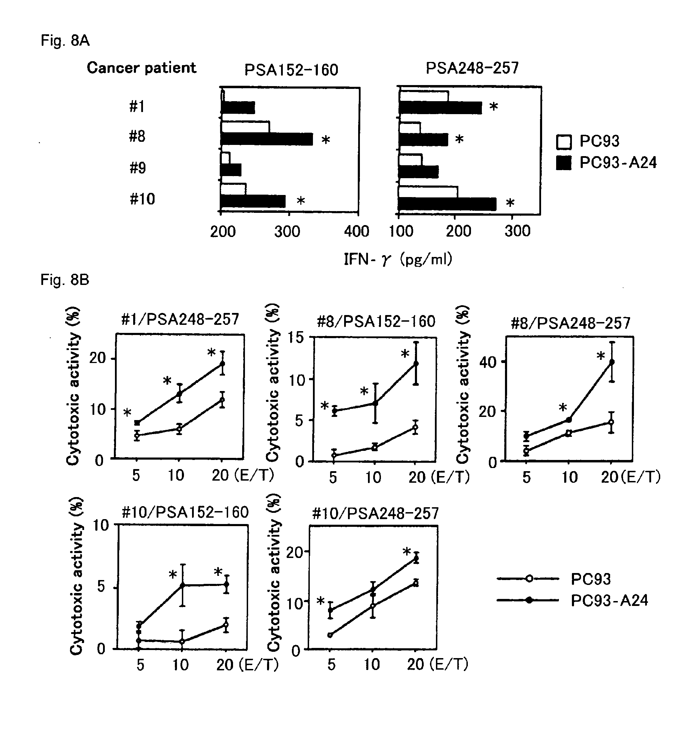

In addition, the same tests were performed on PBMC derived from 4 cases of prostate cancer patients. The results are shown in Fig. 8A and Fig. 8B. The PBMC of the prostate cancer patients, which were stimulated with either PSA152-160 (SEQ ID NO: 1) or PSA248-257 (SEQ ID NO: 2), produced high levels of IFN-γ in response to PC93-A24 cells with significance in 2 out of 4 cases and 3 out of 4 cases, respectively (Fig. 8A). In addition, the PBMC derived from prostate cancer, which were stimulated with PSA152-160 (SEQ ID NO: 1) or PSA248-257 (SEQ ID NO: 2), exhibited cytotoxic activity against PC93-A24 cells and lysed these cells. The cytotoxic activity was significant in comparison to activity against PC93 cells (Fig. 8B).

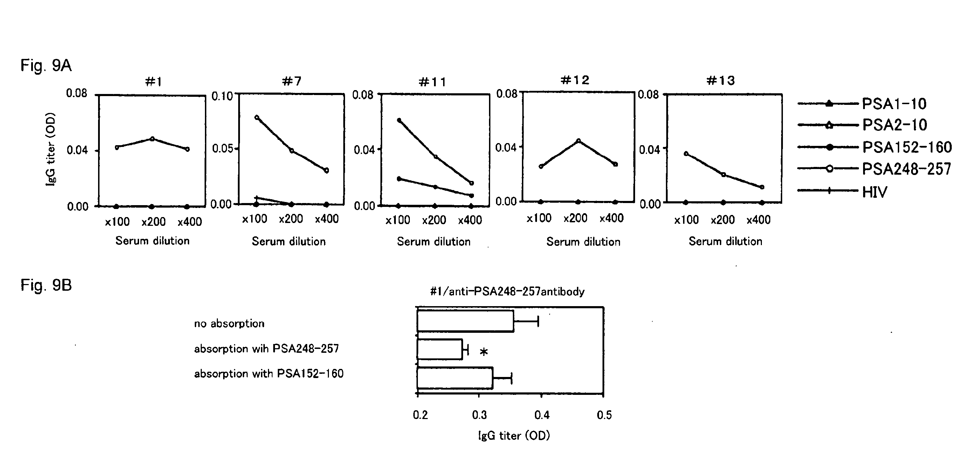

In addition, antibodies against PSA152-160 (SEQ ID NO: 1) or PSA248-257 (SEQ ID NO: 2) were measured in the sera of prostate cancer patients (see Example 3). Antibodies against PSA248-257 (SEQ ID NO: 2) were detected in 5 cases (Fig. 9A). Among these cases, antibodies against PSA152-160 (SEQ ID NO: 1) were also detected in one case. The antibodies against PSA248-257 (SEQ ID NO: 2) were adsorbed by incubating the antibodies in a plate in which PSA248-257 (SEQ ID NO: 2) was immobilized (Fig. 9B); however, the antibodies were not adsorbed by using a plate in which PSA152-160 (SEQ ID NO: 1) was immobilized. These results demonstrate that the detected antibodies are specific to PSA248-257 (SEQ ID NO: 2).

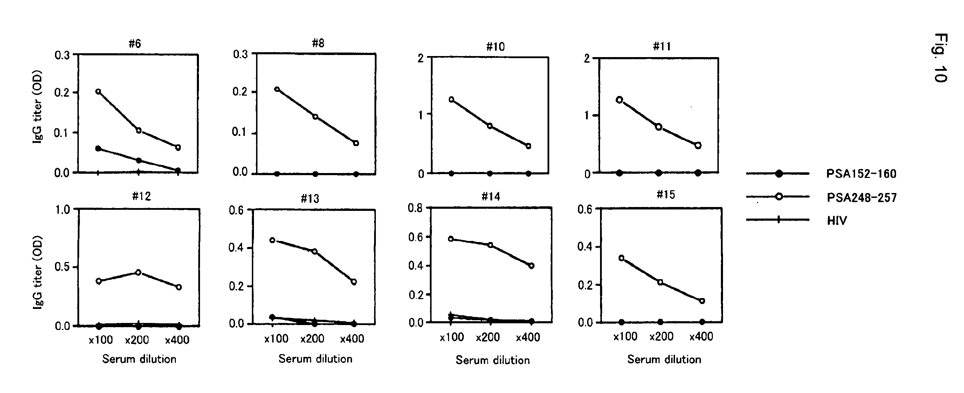

The antibodies against PSA152-160 (SEQ ID NO: 1) and PSA248-257 (SEQ ID NO: 2) were also detected in the sera of healthy subjects. Eight exemplary cases are shown in Fig. 10.

From the foregoing results, it is shown that PSA152-160 (SEQ ID NO: 1) and PSA248-257 (SEQ ID NO: 2) induce CTL from the PBMC derived from HLA-24⁺ healthy subjects and HLA-24⁺ prostate cancer patients. The induced CTL recognize HLA-24⁺ prostate cancer cells and exhibit cytotoxic activity against these cells. In addition, it is shown that specific antibodies against these peptides are present in the sera of prostate cancer patients.

### Example 5

### (Examination of the ability of PSM624-632 (SEQ ID NO: 3) to induce cytotoxic T-lymphocyte)

The PBMC prepared from an HLA-A24⁺ healthy subject and HLA-A24⁺ prostate cancer patient were incubated with PSM624-632 (SEQ ID NO: 3), and then the production of IFN-γ upon the recognition of the target cell, was examined. The PBMC were used after curturing with 10 µg/ml PSM624-632 (SEQ ID NO: 3) for 12 days or 10 days by the same method as in Example 1. C1R-A2401 cells (HLA-A24⁺), pulsed with peptides (10 µg/ml) by the same method as in Example 1, were used as target cells. The PBMC and target cells were mixed and incubated as in Example 1, and the amount of IFN-γ in the culture supernatant was measured.

The results show that PSM624-632 (SEQ ID NO: 3) enhanced the production of IFN-γ from the PBMC in 1 out of 2 cases of prostate cancer patients. Taken together with the results from the separate cases in Example 1, PSM624-632 (SEQ ID NO: 3) enhanced the production of IFN-γ from the PBMC in 6 out of 16 cases. In addition, this peptide enhanced the production of IFN-γ from the PBMC in 3 out of 5 cases of healthy subjects.

Next, using PSM624-632 (SEQ ID NO: 3), induction of CTL that recognize prostate cancer cells and exhibit cytotoxic activity was examined from the PBMC derived from 7 cases of prostate cancer patients other than the cases used in the aforementioned tests. The PBMC were co-cultured with 10 µg/ml of PSM624-632 (SEQ ID NO: 3) for 12 days as described above. Prostate cancer cell PC93-A24, which were created in Example 2 and used as target cells, were incubated with the aforementioned cultured PBMC, and then the amount of IFN-γ in the culture supernatant was measured (see Example 1).

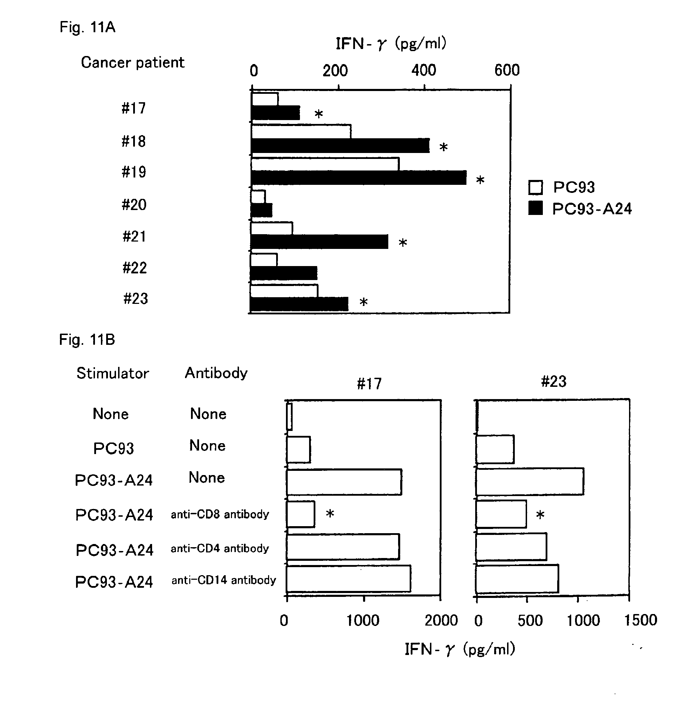

The results are shown in Figs. 11A and 11B. In 5 out of 7 cases, PSM624-632 (SEQ ID NO: 3) enhanced the production of IFN-γ from the PBMC derived from prostate cancer patients in reaction to PC93-A24 cells, with significance (Fig. 11A). The production of IFN-γ in accordance with the induction of the CTL, which were stimulated with PSM624-632 (SEQ ID NO: 3), was inhibited by an anti-CD8 antibody, but was not inhibited by an anti-CD4 antibody or an anti-CD14 antibody (Fig. 11 B). From the foregoing, it is shown that PSM624-632 (SEQ ID NO: 3) induces CTL, which recognize an HLA-24⁺ prostate cancer cell, from the PBMC derived from an HLA-A24⁺ prostate cancer patient.

In addition, antibodies against PSM624-632 (SEQ ID NO: 3) were measured in the sera of prostate cancer patients (see Example 3). Three exemplary cases in which the antibody was detected are shown in Fig. 12A. The antibodies in the sera of the patients were adsorbed by pre-incubation with cellulose beads coated with PSM624-632 (SEQ ID NO: 3) (Fig. 12B). This demonstrates that the antibodies detected in the sera of the patients are specific to PSM624-632 (SEQ ID NO: 3). Furthermore, when the antibodies bound to the beads coated with PSM624-632 (SEQ ID NO: 3) were eluted with a buffered solution containing 0.2 M citric acid and 0.5 M NaCl, antibodies specific to PSM624-632 (SEQ ID NO: 3) were detected in the eluate obtained (Fig. 12C).

From these results, it is shown that PSM624-632 (SEQ ID NO: 3) induces CTL, which recognize HLA-24⁺ prostate cancer cells and exhibit cytotoxic activity against the cells, from the PBMC derived from an HLA-24⁺ healthy subject and HLA-24⁺ prostate cancer patients. In addition, it is demonstrated that specific antibodies against this peptide is present in the sera of healthy subjects and prostate cancer patients.

### Example 6

### (Identification of peptide recognized by HLA-A2-restricted CTL)

In order to obtain a peptide that binds to an HLA-A2 molecule, first, a peptide having an HLA-A2-binding motif was searched for, using the Internet home page <http://bimas.dcrt.nih.gov//molbio/hla_bind/>. Then, 9-mer or 10-mer peptides that were conformable to the motifs were designed based on the amino acid sequence of PSCA and synthesized by a method well-known in the art. The purity of all the synthesized peptides was 90% or greater.

The ability of the synthesized peptide to induce HLA-A2-restricted CTL from the PBMC prepared from an HLA-A2⁺ healthy subject and HLA-A2⁺ prostate cancer patient was examined. The PBMC were prepared from blood by a method of the art; 1x10⁵ PBMC were incubated with 10 µM of peptides in each well of a U-shaped 96-well micro-culture plate (Nunc) using the identical media to that used in Example 1 at 37°C under conditions of 5% CO²-95% air. At 5 days and 10 days of culturing, half of culture medium was removed and replaced with a fresh culture medium containing the peptide equivalent to the peptide that was added in the beginning. After collecting the cells at 15 days of culturing, they were washed and used as effector cells.

Meanwhile, each synthesized peptide (10 µg/ml) was incubated with T2 cells (HLA-A2⁺) for 2 hours at 37°C under conditions of 5% CO₂-95% air, to make the peptide of interest bind to the HLA-A2 molecule expressed on the cell surface. The T2 cells thus pulsed with each of peptide were used as target cells.

Effector cells and 1×10⁴ target cells were mixed at various ratios and incubated for 18 hours at 37°C under conditions of 5% CO₂-95% air. After incubation, 100 µl of supernatant was collected, and the amount of IFN-γ produced was measured by ELISA. In so doing, the effector cells were paired with the target cells such that the peptide used to induce the effector cells and the peptide pulsed to T2 cells were the same. In addition, an HIV peptide was used as a control, and the quantity of IFN-γ produced by CTL that recognized T2 cells pulsed with the HIV peptide was taken as a background and subtracted from each measurement value. Each measurement value (NET) that has been calculated was statistically processed by the Student's t-test, and those with a P value of 0.05 or below were considered to be significant. In addition, a peptide that enhanced the IFN-γ production with significance by 80 pg/ml NET or more was assessed as positive.

As a result, PSCA21-30 (SEQ ID NO: 7) enhanced the production of IFN-γ from the PBMC in 3 out of 5 cases of HLA-A2⁺ healthy subjects. In addition, PSCA7-15 (SEQ ID NO: 6) and PSCA21-30 (SEQ ID NO: 7) enhanced the production of IFN-γ from the PBMC in 5 and 6, out of 12 cases of HLA-A24⁺ prostate cancer patients respectively. That is to say, it was revealed that these peptides induced HLA-A2-restricted CTL from the PBMC derived from HLA-A2⁺ healthy subjects and HLA-A2⁺ prostate cancer patients.

### Example 7

### (Cytotoxic activity of CTL induced by peptides)

The cytotoxic activity of CTL that were induced from the PBMC derived from an HLA-A2⁺ healthy subject or HLA-A2⁺ prostate cancer patient by PSCA7-15 (SEQ ID NO: 6) and PSCA21-30 (SEQ ID NO: 7) were examined in the IFN-γ production test and the ⁵¹Cr release test using prostate cancer cells.

First, the PBMC derived from two healthy subjects and one prostate cancer patient were stimulated with peptides, as in Example 6, prior to use as effector cells. PC93-A2 cells resulting from the introduction and expression of the HLA-A2 gene in HLA-A2⁻ prostate cancer cells PC93 by methods known in the art were used as a target cells. In addition, the HLA-A2⁻ prostate cancer cells were used as controls. A portion of the effector cells was mixed and cultured with target cells. Culturing was performed for 18 hours at 37°C under conditions of 5% CO2-95% air. After incubation, 100 µl of supernatant was collected, and the amount of IFN-γ produced was measured by ELISA.

The results are shown in Table. 2. When incubating the HLA-A2⁺ PBMC derived from the healthy subjects with PSCA21-30 (SEQ ID NO: 7), the production of IFN-γ from the PBMC in response to HLA-A2⁺ prostate cancer cells was also enhanced. In addition, when incubating HLA-A2⁺ PBMC derived from the prostate cancer patient with PSCA7-15 (SEQ ID NO: 6), the production of IFN-γ from the PBMC in response to HLA-A2⁺ prostate cancer cells was enhanced. Statistically significance was observed by the Student's t-test for both results.

**Table 2**

| PBMC donor | Peptide | IFN-γ production (pg/ml) | |
|---|---|---|---|
| | | HLA-A2⁻ prostate cancer cell | HLA-A2⁺ prostate cancer cell |
| HLA-A2⁺ Healthy subject 1 | PSCA21-30 (SEQ ID NO: 7) | 730.8±249.3 | 1150.0±55.4 |
| HLA-A2⁺ Healthy subject 2 | PSCA21-30 (SEQ ID NO: 7) | 1534.8±284.1 | 2297.5±374.7 |
| HLA-A2⁺ Prostate cancer patient 3 | PSCA7-15 (SEQ ID NO: 6) | 490.9±26.2 | 633.3±81.7 |

Next, the PBMC derived from the prostate cancer patient were stimulated by incubating with PSCA7-15 (SEQ ID NO: 6) for 15 days, and then cultured for an additional 10 days for use as effector cells. The cytotoxic activity against prostate cancer cells was then examined. PC93-A2 were used as prostate cancer cells. PC93 were used as a control. The cytotoxic activity was measured by a standard 6-hour ⁵¹Cr release test at an E/T ratio of 20:1 or 40:1, and the obtained results were expressed as the percentages of specific lysis.

As a result, the PBMC derived from the prostate cancer patient, which were stimulated with PSCA7-15 (SEQ ID NO: 6), specifically lysed prostate cancer PC93-A2 cells that expressed HLA-A2 (Table 3).

**Table 3**

| PBMC donors | Cytotoxic activity (% lysis) | | | |
|---|---|---|---|---|
| | prostate HLA-A2⁻ prostate cancer cell | | HLA-A2⁺ prostate cancer cell | |
| | E/T=10 | E/T=20 | E/T=10 | E/T=20 |
| HLA-A2⁺ Prostate cancer patient 3 | 0 | 0 | 2 | 12 |

This makes it clear that PSCA7-15 (SEQ ID NO: 6) induces tumor-specific cytotoxic T-lymphocytes from the PBMC of cancer patients, which recognize tumor cells in an HLA-A2-restricted manner and exhibit cytotoxicity. In addition, since the production of IFN-γ from the PBMC of cancer patients in response to tumor cells is enhanced by PSCA21-30 (SEQ ID NO: 7), it can be inferred similarly that PSCA21-30 (SEQ ID NO: 7) also induces tumor-specific cytotoxic T-lymphocytes that recognize tumor cells in an HLA-A2-restricted manner and exhibit cytotoxicity.

### Example 8

The presence of IgG anti-peptide antibodies against a peptide that is derived from PSCA and has an HLA-A2-binding motif, was measured in the sera of HLA-A2⁺ healthy subjects and in the sera of HLA-A2⁺ prostate cancer patients by ELISA as in Example 3. As a result, antibodies against PSCA21-30 (SEQ ID NO: 7), which is a peptide that induced HLA-A2-restricted CTL from the PBMC derived from prostate cancer patients, were detected in 5 out of 5 cases of healthy subjects and 9 out of 12 cases of prostate cancer patients (Table 4). Antibodies against PSCA7-15 (SEQ ID NO: 6) were detected in none of 5 cases of healthy subjects nor 12 cases of prostate cancer patients.

### Possibilities for Industrial Use

In the present invention, peptides were designed and synthesized based on the amino acid sequences of PSA, PSM, and PSCA, which are known as markers of prostate cancer, and peptides that induce HLA-A2-restricted or HLA-A24-restricted CTL from the PBMC derived from a patient with prostate cancer were identified therefrom. The HLA-A2 allele is found in approximately 40% of Japanese, approximately 53% of Chinese, approximately 49% of North American Caucasians, approximately 38% of South American Caucasians, and approximately 23% of African Blacks. The HLA-A24 allele is found in approximately 60% of the Japanese population (for the most part, 95% of its genotype is A2402), approximately 20% of Caucasians, and approximately 12% of Africans. Therefore, the peptides of the present invention can be applied to specific immunotherapy in a relatively large number of cancer patients. That is to say, the peptides of the present invention provide a highly useful means in the treatment of cancer, for example, prostate cancer. In addition, the peptides of the invention are highly useful, since they can induce and/or activate CTL, which are a cell population comprising plural cells that recognize several types of tumor antigens, and respond to a diversity of cancers by using tumor antigen peptides that have been already reported.

### SEQUENCE LISTING FREE TEXT

Sequence Listing SEQ ID NO: 1: Designed peptide based on the amino acid sequence of PSA, which is recognized by HLA-A24-restricted cytotoxic T-lymphocytes.

Sequence Listing SEQ ID NO: 2: Designed peptide based on the amino acid sequence of PSA, which is recognized by HLA-A24-restricted cytotoxic T-lymphocytes.

Sequence Listing SEQ ID NO: 3: Designed peptide based on the amino acid sequence of PSMA, which is recognized by HLA-A24-restricted cytotoxic T-lymphocytes.

Sequence Listing SEQ ID NO: 4: Designed peptide based on the amino acid sequence of PSCA, which is recognized by HLA-A24-restricted cytotoxic T-lymphocytes.

Sequence Listing SEQ ID NO: 5: Designed peptide based on the amino acid sequence of PSCA, which is recognized by HLA-A24-restricted cytotoxic T-lymphocytes.

Sequence Listing SEQ ID NO: 6: Designed peptide based on the amino acid sequence of PSCA, which is recognized by HLA-A2-restricted cytotoxic T-lymphocytes.

Sequence Listing SEQ ID NO: 7: Designed peptide based on the amino acid sequence of PSCA, which is recognized by HLA-A2-restricted cytotoxic T-lymphocytes.

Sequence Listing SEQ ID NO: 8: Designed peptide based on the amino acid sequence of PSA, which is recognized by HLA-A2-restricted cytotoxic T-lymphocytes.

Sequence Listing SEQ ID NO: 9: Designed peptide based on the amino acid sequence of PSA, which is recognized by HLA-A2-restricted cytotoxic T-lymphocytes.

Sequence Listing SEQ ID NO: 10: oligoDesigned peptide based on the amino acid sequence of PSA, which is recognized by HLA-A2-restricted cytotoxic T-lymphocytes.

Sequence Listing SEQ ID NO: 11: Designed peptide based on the amino acid sequence of PSMA, which is recognized by HLA-A2-restricted cytotoxic T-lymphocytes.

Sequence Listing SEQ ID NO: 12: Designed peptide based on the amino acid sequence of PSMA, which is recognized by HLA-A2-restricted cytotoxic T-lymphocytes.

## Claims

1. A peptide having the amino acid sequence set forth in SEQ ID NO: 7 in the Sequence Listing.

2. The peptide according to claim 1, wherein said peptide is recognised by a cytotoxic T-lymphocyte, and/or the peptide induces a cytotoxic T-lymphocyte.

3. The peptide according to claim 1 or 2, wherein said peptide is recognised by a cytotoxic T-lymphocyte in an HLA-A2-restricted manner, and/or the peptide induces a cytotoxic T-lymphocyte in an HLA-A2-restricted manner.

4. The peptide according to any of claims 1 to 3, wherein said peptide can induce a cytotoxic T-lymphocyte in blood of a cancer patient.

5. A medicament comprising the peptide having the amino acid sequence set forth in SEQ ID NO: 7 in the Sequence Listing, optionally together with at least one additional peptide having the amino acid sequence set forth in any one of SEQ ID NO: 1 to 6 in the Sequence Listing.

6. The medicament according to claim 5 in the form of a cancer vaccine.

7. Use of the cancer vaccine according to Claim 6 for the treatment of prostate cancer.

8. An *in vitro* method for inducing a cytotoxic T-lymphocyte, comprising using at least one peptide having the amino acid sequence set forth in SEQ ID NO: 7 in the Sequence Listing.

9. The method for inducing a cytotoxic T-lymphocyte according to claim 8, comprising the steps of:
i) incubating an antigen-presenting cell that retains HLA-A2 with a peptide having the amino acid sequence set forth in SEQ ID NO: 7 in the Sequence Listing; and
ii) stimulating a cell population that contains a precursor cell of the cytotoxic T-lymphocyte by using the cells obtained at the step i).

10. An antibody that immunologically recognises a peptide according to any one of Claims 1 to 4.

11. A method for identifying a compound that interacts with the peptide according to any of claims 1 to 4 and/or an HLA-A2 molecule, and at least enhances the recognition of said peptide by an HLA-A2-restricted cytotoxic T-lymphocyte, wherein said method comprises measuring the recognition of said peptide by an HLA-A2-restricted cytotoxic T-lymphocyte in the presence and absence of at least one substance.

12. A pharmaceutical composition for use in cancer therapy that contains at least one substance selected from a group consisting of the peptide according to any one of Claims 1 to 4, the antibody of Claim 10, and the compound identified according to Claim 11.

13. A reagent kit comprising at least one substance selected from a group consisting of the peptide according to any one of Claims 1 to 4 and the antibody of Claim 10.

14. A cytotoxic T-lymphocyte that is induced by the method of claims 8 or 9.

15. The cytotoxic T-lymphocyte according to claim 14, which is derived from a peripheral blood mononuclear cell obtained from a prostate cancer patient.
